# EUROPÄISCHE PATENTSCHRIFT

(11) **EP 3 064 170 B1**
(45) Veröffentlichungstag und Bekanntmachung des Hinweises auf die Patenterteilung: **26.05.2021**
(21) Anmeldenummer: 15157544.6
(22) Anmeldetag: 04.03.2015
(51) Int. Cl.: A61C 13/00, A61C 13/09

(54) **Dentaler Prothesenrohling und Verfahren zur Herstellung einer dentalen Prothese**
Dental prosthesis blank and method of making a dental prosthesis
Ebauche de prothèse dentaire et procédé de fabrication d'une prothèse dentaire

(43) Veröffentlichungstag der Anmeldung: 07.09.2016
(73) Patentinhaber: Ivoclar Vivadent AG, 9494 Schaan (LI)
(72) Erfinder: Watzke, Ronny, 6800 Feldkirch (AT); Frei, Christian, 39025 Naturns (IT)
(74) Vertreter: Baronetzky, Klaus

(56) Entgegenhaltungen:
- WO-A1-2010/057584
- WO-A2-2013/068124
- CN-A- 103 654 981
- US-A1- 2013 101 962

## Beschreibung

Die Erfindung betrifft einen dentalen Prothesenrohling sowie ein Verfahren zur Herstellung einer dentalen Prothese aus einem Prothesenrohling. Es ist seit langem bekannt, Prothesen, aber auch Zähne, aus mehreren Schichten aufzubauen.

Als Beispiele hierfür seien die aus der WO 90/13 268 A1 und aus der WO 91/07 141 A1 bekannten Lösungen genannt.

Die US 2013/0 101 962 A1 offenbart ein Verfahren zur Herstellung einer Zahnprothse mit einem Grundteil und einer Vielzahl von damit verbundenen Zähnen.

Die WO 2010/057 584 A1 offenbart einen Fräsblock zur Herstellung von Teil- oder Totalprothesen nach dem CAD/CAM-Verfahren.

Ferner offenbart die WO2013/068 124 A1 einen Fräsblock für eine herkömmliche Prothesenbasis, auf der künstlich geformte Zähne vorgesehen sind.

Basierend auf diesen teilweise recht alten Lösungen sind in neuerer Zeit unter Zuhilfenahme der CAD/CAM-Technik auch mehrschichtige Kunststoffelemente vorgeschlagen worden, die der Erstellung von Zähnen und Prothesen zur Bereitstellung von Zähnen und der Prothesenbasis zur Bereitstellung einer fertigen Prothese dienen sollen. Aufgrund der unterschiedlichen Erfordernisse an die verschiedenen Materialien, nämlich des fleischfarbenen Prothesenbasismaterials einerseits und des zahnfarbenen Zahnmaterials andererseits hat sich dieser Vorschlag bislang nicht durchgesetzt, und zwar auch nicht, obwohl es bereits anderweitig bekannt geworden ist, sowohl Zähne als auch eine Prothesenbasis aus PMMA zu fertigen.

Ferner sind in neuerer Zeit Rapid-Prototyping-Verfahren vorgeschlagen worden, mit dem Ziel, sowohl Zähne als auch Prothesenbasen aus Kunststoffmaterial - oder gegebenenfalls auch aus anderen Materialien - bereitzustellen. Auch diese Entwicklung hat bis jetzt keine rechten Fortschritte erfahren.

Andererseits besteht in Hinblick auf die zunehmende Lebenserwartung eine zunehmende Nachfrage nach Voll- und Teilprothesen, wobei hier der Einfachheit halber unter dem Begriff Prothesen sowohl Voll- als auch Teilprothesen subsumiert werden. Dieser zunehmende Bedarf ist gepaart mit einem ebenfalls zunehmenden Kostendruck, bereits aufgrund der zu erwartenden sich erheblich steigernden Nachfrage.

Daher liegt der Erfindung die Aufgabe zugrunde, einen Prothesenrohling gemäß dem Oberbegriff von Anspruch 1 bzw. ein Verfahren zur Herstellung einer dentalen Prothese aus einem Prothesenrohling gemäß dem Oberbegriff von Anspruch 11 zu schaffen, die zum einen preisgünstig zu fertigen ist, zum anderen eine erheblich verbesserte Akzeptanz aufweist und auch hinsichtlich der Lagerhaltungsmöglichkeiten optimiert ist.

Diese Aufgabe wird erfindungsgemäß durch Anspruch 1 bzw. 11 gelöst. Vorteilhafte Weiterbildungen ergeben sich aus den Unteransprüchen.

Erfindungsgemäß ist es vorgesehen, dass bei einer Zahnprothese, die in einer Ausführungsform einteilig oder einstückig, und in einer anderen Ausführungsform zweiteilig oder zweistückig hergestellt ist, die beiden Materialien in einer speziellen Form miteinander zu verbinden. Die Form ist über die Erstreckung des Zahnfleischbogens der Prothese betrachtet wellenförmig, wobei hier unter wellenförmig auch eine asymmetrische Welle zu verstehen ist, also eine solche, bei welcher ähnlich einer Kettenlinie kleine und enge Bögen sich mit tiefen und weiten Bögen abwechseln.

Ein erfindungsgemäß besonderer Vorteil liegt darin begründet, dass die Wellenform der Prothese von einem zentralen Punkt dieser aus betrachtet strahlenförmig erstreckt. Jedes Wellental und jeder Wellenberg erstreckt sich insofern auf der gleichen Höhe, oder aber, in einer modifizierten Ausführungform auf einer geraden Linie, die gegenüber der Horizontalen um wenige Grade abweichen kann, beispielsweise um 10° nach oben oder nach unten.

Erfindungsgemäß ist es besonders günstig, dass die Zähne des Zahnfleischbogens miteinander verbunden bleiben, aber dennoch aufgrund der aus vestibulärer Richtung betrachtet tiefen Nuten zwischen den Zähnen den Eindruck von Einzelzähnen erwecken. Dies liegt unter anderem darin begründet, dass gerade im Frontzahnbereich seitlich einfallendes Licht Schatten wirft, so dass die Zahnzwischenräume von mindestens einem benachbarten Zahn abgeschattet werden und insofern der erfindungsgemäße Multizahnbogen nicht von Einzelzähnen unterscheidbar ist.

Das fleischfarbene und das zahnfarbene Material werden zur Erstellung des erfindungsgemäßen zweifarbigen Rohling - sei es durch einstückige Herstellung, sei es durch Kleben oder durch Polymerisieren - intensiv miteinander verbunden. Die einstückige und untrennbare Herstellung kann z.B. dadurch erzielt werden, dass die Materialien in einem an der Grenzfläche noch weichen - oder gar flüssigen - Zustand aufeinandergepresst werden, so dass sie - mikroskopisch betrachtet - ineinander eindringen. Der Übergangsbereich ist aber auch bei dieser Art der Herstellung im Submiliimeterbereich, z.B. weniger als 100 µm dick.

Auch beim Aufpolymerisieren besteht ein derartiger Übergangsbereich in der gleichen Größenordnung, und beim Kleben kann die Klebefuge eine geringe Stärke zwischen z.B. 40 und 200 µm aufweisen.

Bevorzugt ist der Rohling des fleischfarbenen Materials mindestens teilweise kreisbogenförmig. Als besonders günstig hat sich die Realisierung über eine Scheibe herausgestellt, wobei sich beispielsweise in einer kreisrunden Scheibe mit einem Durchmesser von 98,5 mm ohne weiteres zwei Prothesenbasen unterbringen lassen.

Das zahnfarbene Material des Rohlings weist im Rohlingzustand eine Größe auf, die größer als ein menschlicher Zahnbogen ist, bevorzugt also größer als 8 cm.

In gleicher Weise lässt sich der Materialaufwand für die Herstellung des zahnfarbenen Rohlings optimieren. Bei Vollprothetik lässt sich beispielsweise in einer kreisförmigen Scheibe mit dem gleichen Durchmesser eine Mehrzahl von Zahnbögen unterbringen. Es ist zum Beispiel möglich, zwei recht große Zahnbögen in an sich bekannter Weise einander gegenüberliegend zu realisieren. Im Falle von Unterkieferprothesen können die Bögen im Wesentlichen als Parabeln realisiert sein, und im Falle von Oberkieferprothesen in Form von Ellipsen.

Erfindungsgmäß ist es günstig, dass die Wellenberge des zahnfarbenen Materials an Zahnpositionen und die Wellentäler an Zahnzwischenraumpositionen vorgesehen sind. Damit ergibt sich nach dem Fertigstellen, z.B. duch gemeinsames Fräsen, eine dem natürlichen Gingivasaum entsprechende Anmutung.

So lassen sich bei optimaler Materialausnutzung aus einer zahnfarbenen Rohlingscheibe z. B. bis zu sechs Zahnbögen fräsen, wobei es sich versteht, dass bei Realisierung von Teilprothesen diese Anzahl sich noch wesentlich erhöhen lässt. Auch ist es möglich, an der Oberseite eines fleischfarbenen Prothesen-Rohlings einen Zahnbogen-Rohling anzubringen, und an der Unterseite - um 180 Grad versetzt - einen weiteren, so dass OK- und UK-Prothesen in einem Zuge herstellbar sind.

In einer weiteren vorteilhaften Ausgestaltung ist es vorgesehen, in den fleischfarbenen Prothesenzahnträger einer Verstärkung zu integrieren. Diese kann beispielsweise aus Metall bestehen, oder aus einem beliebigen anderen geeigneten Material, dessen Festigkeit größer ist als die Festigkeit des fleischfarbenen Materials. Die Verstärkung kann entweder bereits vorab integriert sein, also in beispielsweise in dem fleischfarbenen Rohling, oder unter Ausbildung einer Tasche nachträglich eingebracht, insbesondere eingeschoben werden.

Erfindungsgemäß ist es vorgesehen, in der bevorzugten Ausgestaltung den zahnfarbenen Rohling und den fleischfarbenen Rohling intensiv miteinander zu verbinden - sei es durch Polymerisieren, sei es durch Kleben - und in diesem Zustand die erforderliche Fräsbearbeitung vorzunehmen. Dies hat den Vorteil, dass die Fräsarbeiten wesentlich schneller vonstattengehen können. Ein Einspannen ist lediglich im Bereich der fleischfarbenen Rohlingsscheibe erforderlich. Aufgrund der intensiven Verbindung zwischen dem zahnfarbenen und dem fleischfarbenen Rohling und aufgrund des Formschlusses zwischen diesen mit der kombinierten Wellen- und Strahlenform reicht die Festigkeit der Verbindung überraschend auch für die Fräsbearbeitung und die dort eingebrachten Kräfte aus.

Dies ist zudem gleichsam ein Test für die Belastbarkeit der Verbindung in Hinblick auf die später auszuübenden Scherkräfte während der Mastikation.

Hierbei kommt besonders günstig die Zahnbogenform der zahnfarbenen Masse zur Geltung, die hinsichtlich der Festigkeit der Verbindung einer Alternativlösung mit vereinzelten Zähnen deutlich überlegen ist.

Besonders günstig ist es, dass die Prothese - nach Fixierung des fleischfarbenen und des zahnfarbenen Materials aneinander - durch Fräsen, insbesondere in einer CAD/CAM-Einheit, unter Einbindung der Steuervorrichtung automatisch fertiggestellt werden kann. Mit dieser läßt sich die genaue Lage der vestibulären Grenzlinie der Grenzfläche automatisch und/oder benutzergesteuert festlegen.

In vorteilhafter Ausgestaltung der Erfindung ist es vorgesehen, dass der fleischfarbene Prothesenrohling mit eins bis vier im wesentlichen halbkreisförmigen Wellenbögen für die Aufnahme des zahnfarbenen Materials versehen ist. Jeder dieser Wellenbögen hat zugleich Strahlencharakter.

Bei geschickter Anordnung ist es möglich, dass aus einer Scheibe fleischfarbenen Materials mindestens zwei Prothesen, insbesondere auch unterschiedlicher Größe, hergestellt werden können.

In einer weiteren Ausgestaltung ist es vorgesehen, dass die Grenzfläche in vestibulärer Richtung betrachtet über ihren Verlauf eine weitere, insbesondere weniger stark ausgeprägte Wellenlinie aufweist.

Bevorzugt ist die Wellenform hinsichtlich des genauen Verlaufs an die Gingivalinie des Zahnfleischsaums eines menschlichen Mundes angepasst. Dies gilt in besonderem Maße für den Frontzahnbereich, währen im molaren Bereich auch noch stärker ausgeprägte Wellenberge und Wellentäler möglich sind, um den Formschluss der Verbindung weiter zu verbessern.

Die erfindungsgemäßen Wellenberge und Wellentäler haben insofern eine Doppelfunktion:
Zum einen wird durch den Formschluss zwischen dem Zahnbogen und der Prothesenbasis die Festigkeit deutlich verbessert und insofern die Klebung, die beispielhaft anstelle einer Polymerisation vorgenommen werden kann, unterstützt.

Zum anderen lässt sich hierdurch der Rot/Weiß-Übergang ohne weiteres darstellen, wobei es sich versteht, dass bei der fertiggestellten Prothesen jeder zahnfarbene Wellenberg der Sichtkante des Zahns an seinem Zahnhals gegenüber der von dem fleischfarbenen Material gebildeten Gingiva folgt.

Eine CAD/CAM -Vorrichtung stellt die dentale Prothese fertig, indem sie den genauen Verlauf der Gingivalinie, also der vestibulären Grenzlinie der Grenzfläche aus okklusaler Richtung betrachtet, basierend auf patientenspezifischen Daten festlegt und basierend heirauf die Prothese erzeugt. Damit wird die Größe der Prothese patientenspezifisch festgelegt, und es kann auch unterschiedlichen Zahnbogenformen Rechnung getragen werden, also z.B. eher runden oder eher dreieckförmigen Formen.

Die Bereitstellung der erforderlichen Patientendaten erfolgt folgendermaßen:
Entweder wird zunächst in an sich bekannter Weise ein Intraoralscan durchgeführt, oder es erfolgt eine Abdrucknahme und dann in ebenfalls bekannter Weise ein 3D-Scan dieser. Anatomisch relevante Punkte werden markiert und diene als Referenzpunkte. Der Software der CAD/CAM-Vorrichtung werden die so gewonnenen Daten zugeleitet, und diese erstellt automatisch mit ihrer Steuervorrichtung einen Vorschlag für eine Prothese, eine sogenannte virtuelle Prothese. Diese ist aber vom Benutzer, also z.B. vom Zahntechniker, an der CAD/CAM-Vorrichtung änderbar.

Beispielsweise läßt sich der Verlauf der Gingivalinie - aus Okklusalrichtung betrachtet - ohne weiteres nach ästhetischen Gesichtspunkten anpassen.

Die Steuervorrichtung berücksichtigt hierbei auch die Abmessungen und die Geometrie der Zähne, im Vergleich zu dem Zahnbogen-Rohling und dessen Abmessungen. Wenn sich herausstellt, dass der erwünschte Zahnbogen nicht in dem Rohling Platz finden würde, gibt die Software ein entsprechendes Warnsignal ab.

Gleiches gilt für den Prothesenbasis-Rohling, der auch als Gingivarohling bezeichnet werden kann. Wenn die Größe der Prothesenbasis die des vorgesehenen Rohlings an einer Stelle übersteigt, wird ebenfalls ein Warnsignal abgegeben, mit dem Hinweis, dass der nächstgrößere Rohling verwendet werden sollte.

Liegt die erwünschte Form virtuell fest, werden die gewonnenen Daten für die Weiterbearbeitung freigegeben. Zur subtraktiven Erzeugung der Prothese werden sie einer Fräsmaschine der CAD/CAM-Vorrichtung zugeleitet, in die ein zweifarbiger erfindungsgemäßer Rohling mit wellen/strahlenförmiger Grenzfläche eingespannt ist oder wird.

Im Falle einer generativen Fertigung, welche nicht Teil der vorliegenden Erfindung ist, werden die Daten einer Rapid-Prototyping-Vorrichtung zugeleitet, in der die Form der Zahnbogen-Rohlings und des Prothesenbasis-Rohlings virtuell mit der erfindungsgemäßen strahlen-/wellenförmigen Grenzfläche hinterlegt ist. Basierend auf den gewonnenen patientenspezifischen Daten und der Designfunktion der CAD/CAM-Vorrichtung wird die vestibuläre Grenzlinie der Grenzfläche konstruiert, und die dann die Prothese basierend hierauf erzeugt.

Wesentlich ist es jedenfalls, dass der Zahnbogen-Rohling an der Grenzfläche zum Prothesenbasis-Rohling eine exakte Negativform der Oberfläche des Prothesenbasis-Rohlings hat, so dass die beiden strahlenförmig sich erstreckenden Wellenlinien exakt zueinander passen und ohne weiteres miteinander intensiv verbindbar sind.

Es ist selbstverständlich möglich, beispielsweise auch sogar fünf einzelne Teile herzustellen und dann entsprechend den Bedürfnissen in geeigneter Weise zusammenzufügen oder zu kombinieren, um die Rohlingscheibe für den Kunden bereitszustellen

Der Kunde fräst dann gleichsam aus dem Vollen, also das Zahnmaterial mit dem Gingivamaterial gleichzeitig,
In einer modifizierten Ausgestaltung ist es vorgesehen, eines der Materialien, beispielsweise die Prothesenbasis, hinsichtlich der Grenzfläche zum Zahnbogen hin vorzumodulieren. Bei dieser Ausführungsform wird dann kurzerhand diese Strahlenwellenform als Gußbasis verwendet, und dass der Zahnbogen dort mit einer entsprechenden Form angegossen und dann durchpolymerisiert.

Auch bei dieser Lösung ergibt sich eine exakt vorgegebene Wellenlinie mit dem erwünschten Gingivasaum und einer strahlenförmigen Ausrichtung mit in vestibulärer Richtung breiter werdenen Wellenbergen und Wellentälern.

Durch die Steuervorrichtung läßt sich basierend auf der gewonnenen Patientendaten die individuelle Zahnform, aber auch die Rotation und die Angulation der Zähne festlegen, und ebenso die Form der Prothesenbasis, so dass auch unterschiedliche Designs von Totalprothesen erzeugbar sind.

Durch die Strahlenform ist auch sichergestellt, dass mit zunehmender Breite - in vestibulärer Richtung betrachtet - zugleich auch die Höhe der Zähne und Wellenberge zunimmt. Dies entspricht auch den anatomischen Verhältnissen, denn typischerweise wachsen die Dimensionen von Zähnen mit zunehmender Zahnbreite in allen drei Raumachsen so, dass ein hinterer Zahn (in lingual/vestibulärer Richtung betrachtet) zugleich auch breiter (mesial/distal betrachtet) aber auch höher (okklusal-inzisal/ zervikal betrachtet) ist.

Wenn die Prothesenbasis als Rohling in Scheibenform vorliegt, ist es bevorzugt, in sie halbkreisförmige Wellenbögen einzubringen. Diese dienen der Aufnahme der Zahnbögen, wobei es sich versteht, dass die Zahnbögen zu den Wellenbögen der fleischfarbenen Scheibe passende Wellenbögen aufweisen.

Überraschend ist es hierdurch möglich, ästhetisch besonders gelungene Prothesen auch bei sehr unterschiedlichen Größen der Zähne bereitzustellen, wobei es auch möglich ist, beispielsweise bei Teilprothesen einerseits aus einem zahnfarbenen Rohling einen kleinen Zahnbogen auszubilden, und hieran anschließend oder zugleich an einer anderen Stelle z. B. einen großen Zahnbogen.

Hierbei wird dann der große Zahnbogen auf eine fleischfarbene Prothesenbasis und der andere Zahnbogen auf eine weitere, ebenfalls fleischfarbene Prothesenbasis aufgebracht, und erfindungsgemäß werden an der Grenzschicht die fleischfarbenen und zahnfarbenen Materialien miteinander verbunden.

Die Verbindung kann sowohl durch Kleben als auch durch Anpolymerisieren erfolgen, und aufgrund der Wellenform erfolgt zusätzlich eine formschlüssige Verbindung, die insbesondere seitlich wirkenden Kaukräften entgegenwirkt.

Von den Kaukräften sind insbesondere die Scherkräfte relevant, die bekanntlich über 1000 N betragen können (vergleiche zum Beispiel die Dissertation "Entwicklung und Anwendung einer Methode zur Kaukraftmessung", Tobias Fink, Charite Berlin 2007).

In diesem Zusammenhang ist es besonders günstig und steigert in überraschendem Maße die Dauerhaltbarkeit der Prothese, dass die Formschlussverbindung der Teile der Prothese die auftretenden Wechsellasten besser verträgt.

Erfindungsgemäß sind die Zähne der erfindungsgemäßen Prothese über Brücken aus zahnfarbenen Material unter Bildung des Zahnbogens miteinander verbunden. Hierdurch ist im Vergleich mit Einzelzähnen die seitliche Abstützung gegenüber Scherkräften um eine Größenordnung verbessert.

Dies gilt übrigens auch - wenn auch in etwas vermindertem Maße - für Teilprothesen, die erfindungsgemäß unter Prothesen subsumiert werden.

Während einzeln gefertigte und konfektionierte Zähne typischerweise in Zahnkavitäten in einer gemäß dem Stand der Technik gefertigten Prothesenbasis aufgenommen sind und aufgrund der Hebelwirkung der Kaukräfte bei der Mastikation erheblichen Scherbelastungen unterworfen sind, die auch die Klebefläche stark belasten, ist es erfindungsgemäß vorgesehen, diese Scherkräfte über mindestens zwei Wellenberge und Wellentäler formschlüssig aufzufangen. Erfindungsgemäß ist damit die gefürchtete Hebelwirkung des Einzelzahns mit dem Hebelverhältnis von etwa 2:1 eliminiert, denn der so zur Verfügung gestellte Zahnbogen, der mindestens zwei einander benachbarte Zähne bei Teilprothesen umfasst, hat eine deutlich größere Breite als Höhe, so dass die Hebelwirkung von 2:1 beispielsweise auf 0,5:1 reduziert ist.

Es versteht sich, dass bei vollständigen Zahnbögen, wie sie bei Vollprothesen erforderlich sind, die diesbezügliche Hebelwirkung beispielsweise auf Werte zwischen 0,06:1 und 0,1:1 reduziert ist, entsprechend dem je patientenspezifisch vorgesehenen Höhen-/Breitenverhältnis des zahnfarbenen Materials der erfindungsgemäßen Prothese.

Erfindungsgemäß ist es vorgesehen, dass die Wellenform als Grenzfläche zwischen den Materialien den Gingivasaum simuliert. Dieser ist selbstverständlich von Patient zu Patient unterschiedlich, und erfindungsgemäß ist es insofern besonders günstig, dass in Hinblick auf die Strahlenform in vestibulärer Richtung betrachtet die Größe, also die Breite und die Tiefe der Wellenberge und der Wellentäler außen, also an der Vestibulärseite des Zahnbogens an die Erfordernisse anpassbar ist.

Hierzu wird kurzerhand durch die erfindungsgemäße Steuervorrichtung für die Herstellung der erfindungsgemäßen Prothese die radiale Lage des Zahnbogens - also wiederum die Lage in vestibulärer/palatinal bzw. lingualer Richtung betrachtet - festgelegt und der erwünschte per CAD festgelegte Zahnbogen beispielsweise in einem subtraktiven Verfahren zusammen mit der Prothesenbasis erzeugt. Beispielhaft kann dies in einer Fräsmaschine der CAD/CAM-Vorrichtung erfolgen, wie es vorstehend erwähnt wurde.

Die fleischfarbenen und zahnfarbenen Materialien sind erfindungsgemäß intensiv miteinander verbunden, sei es durch Kleben, sei es durch Polymerisieren, oder aber auch durch eine einstückige Herstellung.

Bei zweistückiger Herstellung ist es günstig, dass die beiden Materialien zu einem beliebigen Zeitpunkt und auch in beliebiger Selektion zueinander passend miteinander verbunden werden können.

Mit halbkreisförmigen Wellenbögenaufnahmen lässt sich der Vollkreis der Scheibe dann optimal ausnutzen. Dies gilt für Vollprothesen, während für Teilprothesen auch kleinere Bogensegmente als halbkreisförmige, also beispielsweise 60°-Segmente oder dergleichen, in Betracht kommen, so dass eine größere Vielzahl von Teilprothesen aus einer einzigen fleischfarbenen Scheibe kostengünstig und rasch herstellbar ist.

Bei einer Vollprothese ist es auch möglich, die Wellenform - über den Zahnfleischbogen betrachtet - nicht exakt horizontal verlaufen zu lassen, sondern entsprechend dem natürlichen Zahnverlauf im Inzisalbereich etwas abzusenken, beispielsweise um 1mm, und dementsprechend im Molarbereich anzuheben, so dass der Speeschen Kurve und der Wilsonkurve Rechnung getragen wird.

Weitere Vorteile, Einzelheiten und Merkmale ergeben sich aus der nachfolgenden Beschreibung mehrerer Ausführungsbeispiele der Erfindung anhand der Zeichnungen.

Es zeigen:
- Fig. 1: eine schematische perspektivische Ansicht eines Rohlings zur Herstellung einer dentalen Prothese, nämlich eines kreisscheibenförmigen fleischfarbenen Rohlings;
- Fig. 2: die Darstellung gemäß Fig. 1 im Schnitt;
- Fig. 3: eine modifizierte Ausführungsform der Rohlings gemäß Fig. 1;
- Fig. 4: eine weitere modifizierte Ausgestaltung eines Zahnbogen-Rohlings für die Bereitstellung einer erfindungsgemäßen dentalen Prothese;
- Fig. 5: eine Ansicht des Prothesenbasis-Rohlings mit eingezeichneten möglichen Grenzlinien der Grenzfläche zur Bildung des Gingivasaumes.;
- Fig. 6: eine weiter modifizierte Ausgestaltung des Zahnbogen-Rohlings gemäß Fig. 4;
- Fig. 7: eine fertige dentale Prothese in einer Ausführungsform im Schnitt;
- Fig. 8: eine weitere Ausführungsform einer Prothese gemäß Fig. 7;
- Fig. 9a: und
- Fig. 9b: weitere Ausführungsform von erfindungsgemäßen dentalen Prothesen in fertiggestelltem Zustand;
- Fig. 10: und
- Fig. 10a: schematische Darstellungen der Rohlinge für die Bereitstellung einer erfindungsgemäßen Prothese, unter Darstellung des zahnfarbenen Rohlings; und
- Fig. 11: eine schematische Darstellung des Rohlings gemäß Fig. 10, wobei verschiedene Zahnbogen-Größen alternativ eingezeichnet sind.

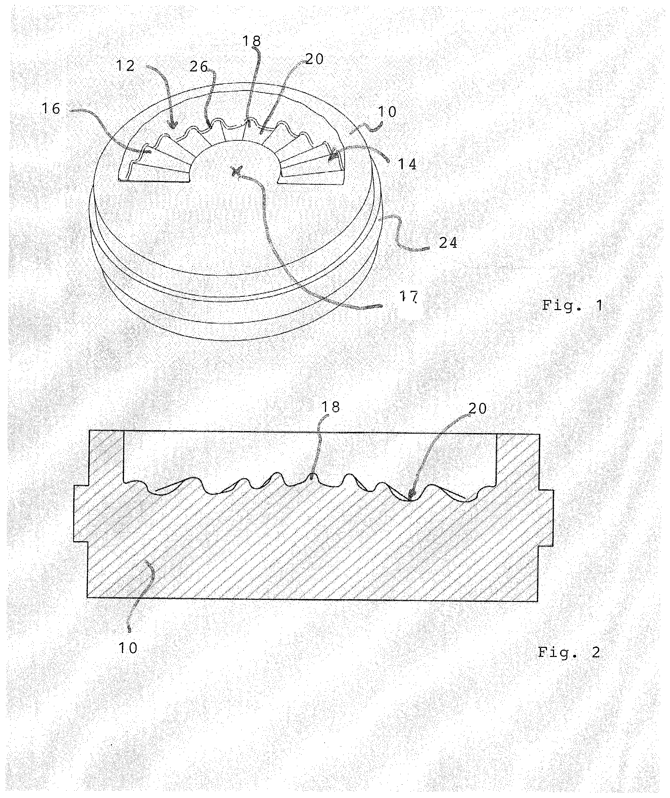

In Fig. 1 ist ein fleischfarbener Rohling 10 als Vorprodukt einer erfindungsgemäßen dentalen Prothese einer Ausführungsform in perspektivischer Ansicht dargestellt. Der Rohling ist vom Grundaufbau her scheibenförmig und besteht aus fleischfarbenem Material, das auch als Gingivamaterial zu bezeichnen ist. Er weist an seiner Oberseite - in der Darstellung gemäß Fig. 1 - eine Ausnehmung 12 auf, die in besonderer Weise ausgebildet ist. Grundsätzlich erstreckt sich die Ausnehmung 12 in Form eines Halbkreises, also bogenförmig. Eine der Besonderheiten des Bogens ist die Wellenform der Unterseite der Ausnehmung 12, die sich in Richtung des Bogens verlaufend erstreckt. In radialer Richtung, also vom Mittelpunkt 17 des Bogens nach außen, also zur vestibulären Seite hin, ist eine Strahlenform vorgesehen.

Der Bogen 16 besteht aus einander abwechselnden Wellenbergen 18 und Wellentälern 20. Diese erstrecken sich winkeltreu, so dass an der gleichen Winkelposition des Bogens 16 der gleiche Wellenberg bzw. an einer anderen, ebenfalls gleichen gleichbleibenden Winkelposition das gleiche Wellental 20 besteht.

Aus Fig. 1 ist ersichtlich, dass die Wellenform 14 des Bogens 16 von einer Sinusform etwas abweicht. Tatsächlich bildet die Wellenform den Gingivasaum nach, und übernimmt insofern teilweise Elemente einer Kettenlinie und teilweise Elemente einer Sinusform.

Die bogenförmige Ausnehmung 12 ist dafür bestimmt, einen passend hierzu geformten Rohling 32 aus zahnfarbenden Material aufzunehmen. Die dem Gingivarohling zugewandte Seite des Rohling aus zahnfarbenen Material weist dementsprechend eine Negativform zur Wellenform 14 am Grund der Ausnehmung 12 auf. Die Form des zahnfarbenen Rohlings 32 passt insofern exakt zur Ausnehmung 12, gegebenenfalls unter Belastung eines Klebespalts mit einer Stärke zwischen 50µm 50 µm und 150 µm.

Dementsprechend lässt sich der zahnfarbene Rohling 32 in die Ausnehmung 12 des Gingivarohlings 10 ohne weiteres einkleben.

Alternativ ist es auch möglich, die Verbindung zwischen diesen Rohlingen durch Polymerisation herzustellen.

Durch das Zusammenfügen erhält der Rohling 10 aufgrund der Befüllung der Ausnehmung 12 insgesamt wiederum die Form einer Kreisscheibe, wobei in an sich bekannter Weise ein Ringvorsprung 24 umlaufend ausgebildet ist und der Lagerung in dem Werkstückhalter einer Dentalfräsmaschine dient.

Hierbei ist es vorgesehen, den Rohling 10 über eine Positioniervorrichtung, die eine verdrehsichere Lagerung ermöglicht, an einer genau vorbestimmten Position zu lagern. Der ggf. teilbearbeitete Rohling ist so zur Überprüfung bei Bedarf zu entnehmen, und kann dann lagerichtig wieder eingesetzt werden.

Erfindungsgemäß wird nun, nachdem die beiden Rohlinge in beschriebener Weise oder in einer beliebigen anderen geeigneten Weise miteinander intensiv verbunden sind, die per CAD/CAM vorgegebene Fräsbearbeitung durchgeführt. Mit dem so erstellten Rohling läßt sich ein beliebiges Design von Prothesen, also Prothesenbasen und Zähnen realisieren. Die komplette dentale Prothese lässt sich in einem Zuge per Fräsbearbeitung herstelllen, und die Gingivalinie 26 ermöglicht es, dass der so bereitgestellte Zahnfleischsaum auf den ersten Blick von einem natürlichen Zahnfleischsaum nicht zu unterscheiden ist.

Auch wenn die Ausnehmung 12 hier als kreisbogenförmig beschrieben ist, versteht es sich, dass beliebige andere Ausgestaltungen der Form möglich sind. Wie es in Fig. 1 angedeutet ist, ist tatsächlich die Wellenform 14 im vestibulär/inzisalen Bereich etwas abgesenkt und im vestibulär/molaren Bereich etwas erhoben, entsprechend dem menschlichen Zahnfleischsaum.

Eine mögliche Verteilung der Wellenberge und Wellentäler ist aus der in Fig. 2 dargestellten Schnittansicht des Gingivarohlings 10 ersichtlich. Durch die Strahlenform der Wellenberge bzw. Wellentäler 18 bzw. 20 entsteht eine asymmetrische Verteilung der Wellenberge und Wellentäler, wie es aus Fig. 2 ersichtlich ist.

Die Bogenbreite des Bogens 16 - in radialer Richtung betrachtet - ist erheblich größer als die vestibulär/linguale bzw. vestibulär/palatinale Erstreckung eines Prothesenzahns. Hierdurch ist es zunächst möglich, eine beliebige Radialposition des per CAD bereitzustellenden Zahnbogens festzulegen. Die Größe der Prothese, aber auch der zugehörigen Zähne, lässt sich daher in weiten Bereichen an die Erfordernisse des menschlichen Kiefer des Patienten anpassen. Durch die Strahlenform entstehen gleichsam automatisch bei einem kleineren Bogen auch kleinere Zähne, denn der Abstand zwischen zwei Bergen 18 bzw. zwischen zwei Tälern 20 entspricht je dem Rastermaß der Zähne.

Erfindungsgemäß besonders günstig ist es, dass die eingebrachten Zähne auch nach dem Fräsen nicht vereinzelt sind, sondern in Form eines Zahnbogens vorliegen. Die Zahnzwischenräume sind bevorzugt tiefliegend ausgebildet und die Materialbrücken zwischen den einzelnen Zähnen sind zur lingual/palatinalen Seite hin verlagert. Hierdurch ist gewährleistet, dass der optische Eindruck des erfindungsgemäßen Zahnbogens sich praktisch nicht von einem Zahnsatz mit Einzelzähnen unterscheiden lässt.

Die Realisierung des Zahnbogens erlaubt zudem eine wesentlich erhöhte Festigkeit. Dies erlaubt es, mit zahnwurzelfreien Zähnen auszukommen, also Zähne, die nur durch die intensive Verbindung mit dem Gingivarohling 10 in Position gehalten werden.

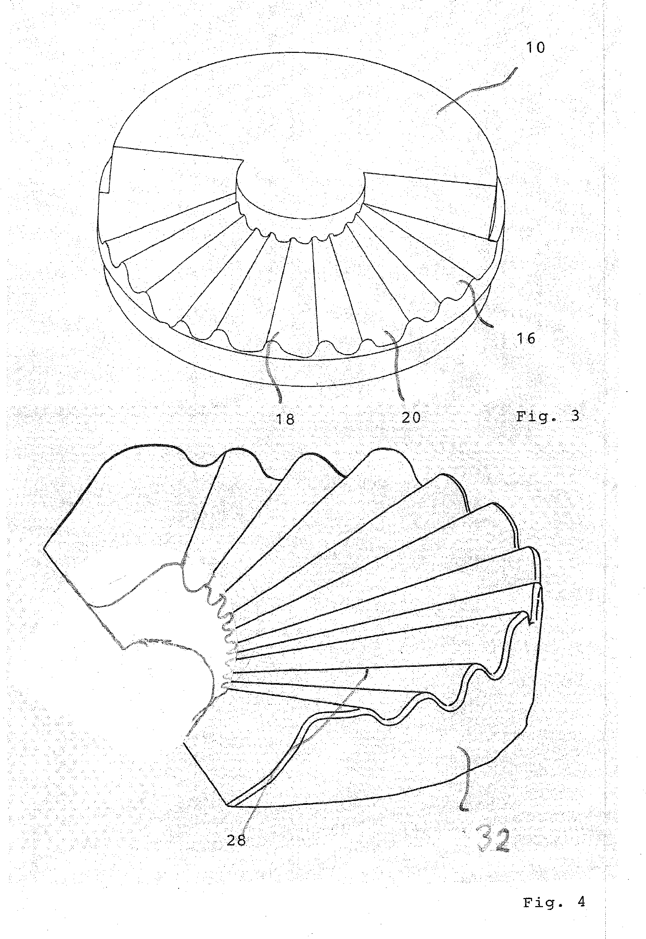

Für die präzise Relativpositionierung des Zahnrohlings 32 gegenüber dem Gingivarohling 10 ist die Realisierung der Ausnehmung 12 günstig, wie es in Fig. 1 und Fig. 2 dargestellt ist. In einer modifizierten Ausgestaltung erstreckt sich der Bogen 16 jedoch bis zum Außenumfang des Gingivarohlings 10, wie es aus Fig. 3 ersichtlich ist. Auch diese Ausführungsform ist geeignet, eine für viele verschiedene Größen und Formen geeignete Prothesenbasis zu bilden, wobei diese Ausführungsform auch noch etwas vergrößerte Zahnbögen ermöglicht.

Aus Fig. 3 ist eine gleichmäßige Verteilung der Wellentäler 20 und der Wellenberge 18 ersichtlich. In einer noch weiter modifizierten Ausgestaltung ist es vorgesehen, die Wellenberge und -Täler im Bereich der Schneidezähne enger und weniger hoch zu realisieren, und dementsprechend im Bereich der Prämolaren und erst recht der Molaren diese breiter und mit größerer Höhe zu realisieren. Dies trägt der unterschiedlichen Zahngröße und Zahnbreite über den Verlauf eines menschlichen Zahnsatzes Rechnung. Typischerweise ist jedoch ein menschlicher Zahnsatz nicht exakt kreisbogenförmig, sondern springt im Inzisalbereich, also insbesondere im Bereich der Zähne I, die eine nahezu ebene Labialfläche aufweisen, gegenüber einem Kreisbogen etwas zurück. Aufgrund der Strahlenform der Berge 18 und Täler 20 ergibt dies dann automatisch dort ein kleineres Rastermaß.

Andererseits sind typischerweise die menschlichen OK-Zähne II etwas schmaler als die OK-Zähne I. Auch dies kann durch entsprechende Anpassung der Breite der Täler und Berge an der betreffenden Winkelstellung berücksichtigt werden, gerade im Falle von Oberkieferprothesen.

Ein demgegenüber noch weiter modifizierter Zahnbogen-Rohling 32 ist aus Fig. 4 ersichtlich. Die dort ersichtliche Form von sich strahlenförmig erstreckenden Wellenbergen und Wellentälern paßt formtreu zum Prothesenbasis-Rohling 10, in die dortige Ausnehmung 12 hinein.

Bei dieser Ausführungsform erstrecken sich die Strahlen 28 zu einem Mittelpunkt, der gegenüber der Höhe der Gingivalinie deutlich abgesenkt ist. Hierdurch entsteht eine Neigung der Strahlen 28 gegenüber der Horizontalen, die bestimmten Ausführungsformen günstig ist und eine bessere Lagerung des Zahnbogens im palatinalen/lingualen Bereich ermöglicht.

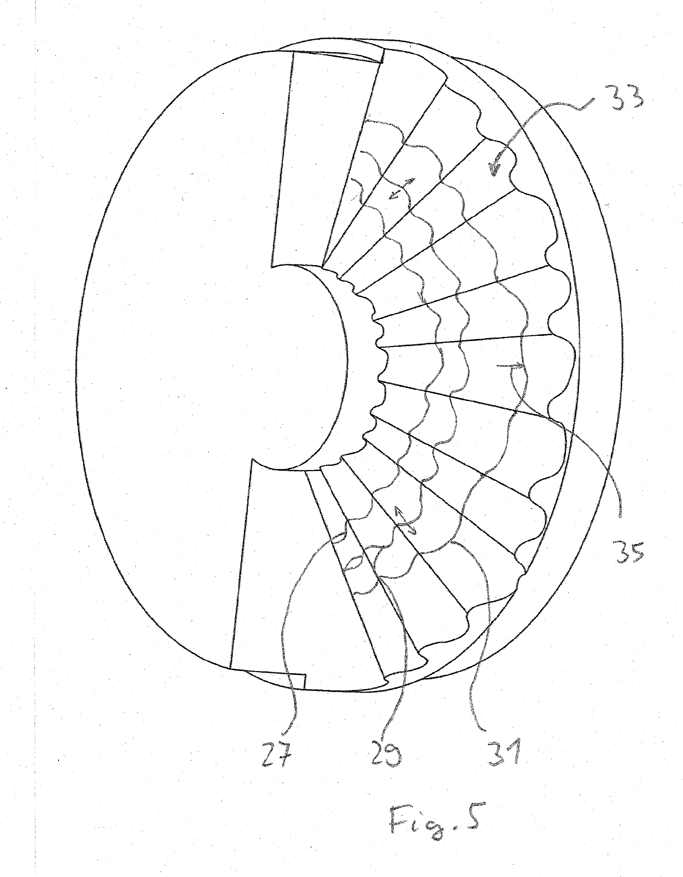

Ein entsprechend frei gelegter teilbearbeiteter Prothesenbasis-Rohling 10 ist aus Fig. 5 ersichtlich, welcher die anhand von Fig. 4 beschriebene Neigung der Strahlenlinie 28 ersichtlich macht. Zudem sind in Fig. 5 verschiedene Grenzlinien 27, 29 und 31 eingezeichnet, die die Fräsgrenze der Grenzfläche 33 gegenüber dem Zahnbogen-Rohlung 32, die insofern als Prothesen-Gingivasaum anzusehen ist, deutlich machen.

Deren Lage läßt sich patientenspezifisch von der Steuervorrichtung einstellen, wobei bei eher dreieckförmigen Kieferstrukturen der mesiale Bereich entsprechend dem Pfeil 35 vorspringt, und auch der Linienverlauf im übrigen in weiten Bereichen an die Erfordernisse anpassbar ist.

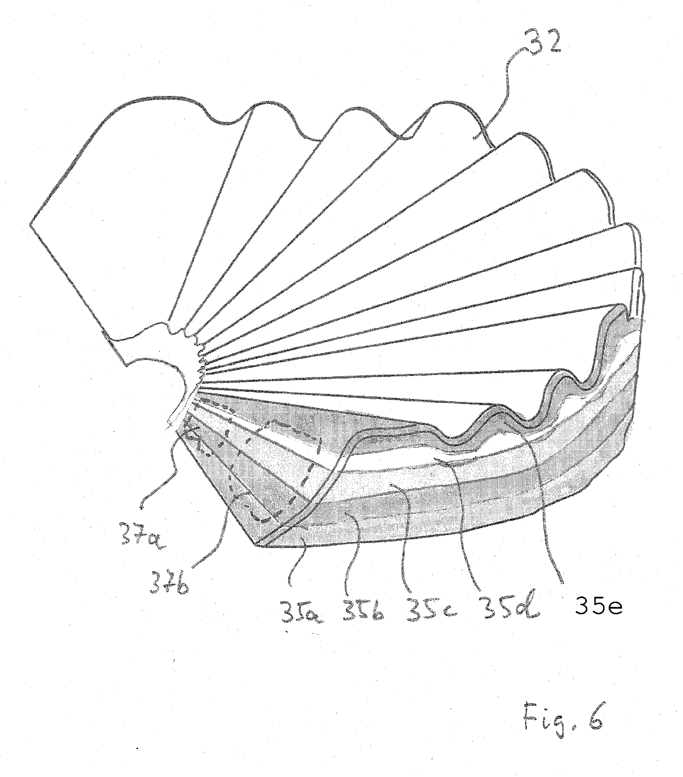

Aus Fig. 6 ist ersichtlich, in welcher Weise bei Bedarf ein Zahnbogen 32, der in einer Kreisbogenvertiefung in dem teilbearbeiteten Prothesenbasis-Rohling 10 abgesenkt gelagert werden soll, geschichtet aufgebaut sein kann.

Schematisch ist ersichtlich, dass der Zahnbogen-Rohling 32 aus 5 Schichten 35a, 35b, 35c, 35d und 35e aufgebaut ist, die zur Grenzfläche 33 hin immer dunkler und opaker werden, entsprechend dem Dentin eines menschlichen Zahnes. Es versteht sich, dass auch deutlich mehr oder auch weniger Schichten eingesetzt werden können.

Der Schichtübergang 35e - 35d folgt hierbei noch abgeschwächt dem Verlauf der Strahlen, Wellenberge und Wellentäler der Grenzfläche, während diese Ausprägung zur Schicht 35a hin immer abgeschwächter wird.

Es versteht sich, dass hier bevorzugt die beiden Rohlinge je separat voneinander per Fräsen fertiggestellt werden und dann aneinandergefügt und durch Kleben intensiv miteinander verbunden sind. Die Seitenflanken der dortigen Kreisbogenvertiefung bilden beim Fräsen gleichermaßen Anschläge des erfindungsgemäßen Zahnbogens in mesial/distaler Richtung. Dies erlaubt eine besonders gute Verankerung des Zahnbogens, wie es gerade bei vergleichsweise kleinen Prothesen wichtig ist.

Durch Abfräsen der Außenseite des Prothesenbasis-Rohlings 10 lässt sich wiederum die erfindungsgemäß besonders günstige Zahnfleischsaumlinie zwischen rotem und weißen Material sichtbar machen.

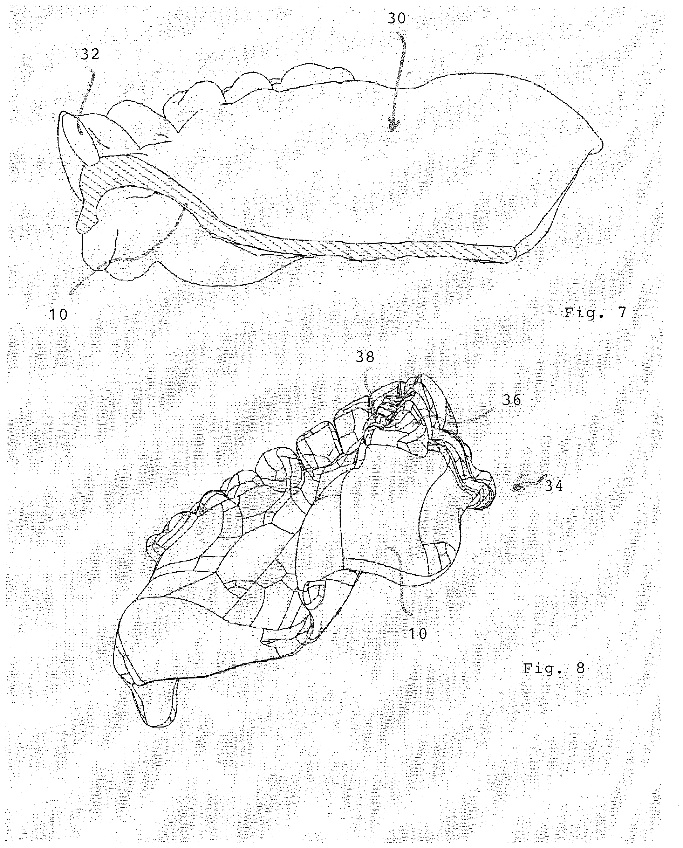

Aus Fig. 7 ist ersichtlich, in welcher Weise eine fertige Prothese 30 im Schnitt ausgebildet sein kann.

Es ist ersichtlich, dass sich das zahnfarbende Material 32 entsprechend dem Gingivasaum, jedoch ohne das fleischfarbene Material 10 zu durchtreten, entsprechend dem natürlichen Zahnbogen an der so gebildeten Prothesenbasis entlang erstreckt. Es ist auch ersichtlich, dass in dieser bevorzugten Ausgestaltung das Material 32 auch in radialer Richtung formschlüssig in dem Material 10 gehalten ist.

Aus Fig. 8 ist die fertige dentale Prothese 34 unter Zuhilfenahme eines Drahtmodells ersichtlich. Anhand des hintersten Molaren 36 als Teil des erfindungsgemäßen Zahnbogens 38 erkennt man, dass dieser intensiv in Gingivamaterial eingebettet, bzw. intensiv mit diesem verbunden ist.

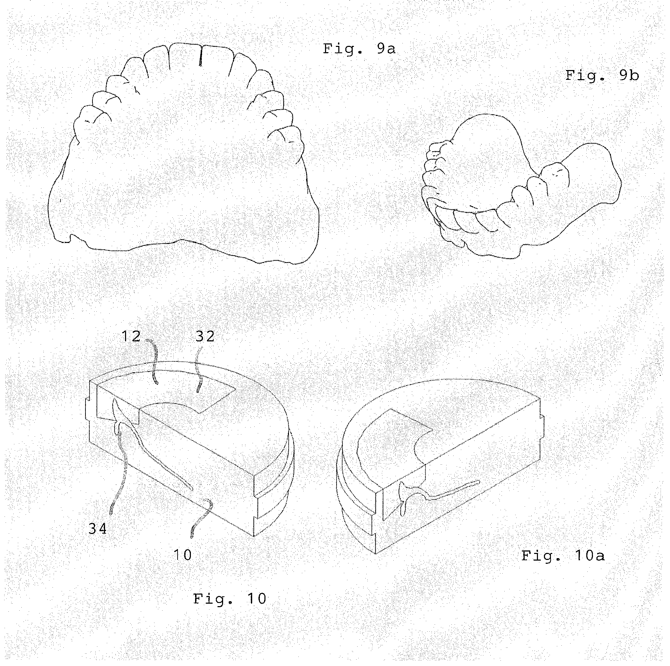

Aus Fig. 9a und 9b sind entsprechend hergestellte Prothesen in unterschiedlichen Blickrichtungen perspektivisch ersichtlich.

Aus Fig. 10 und Fig. 10a ist ersichtlich, in welcher Weise das bogenförmige zahnfarbene Material 32 - in dieser Darstellung noch in Rohlingform - in die Ausnehmung 12 des Gingivarohlings 10 eingebracht ist und dort aufgenommen wird. Schematisch ist je auch die spätere Form der Prothese 34 in Fig. 10 und 10a dargestellt.

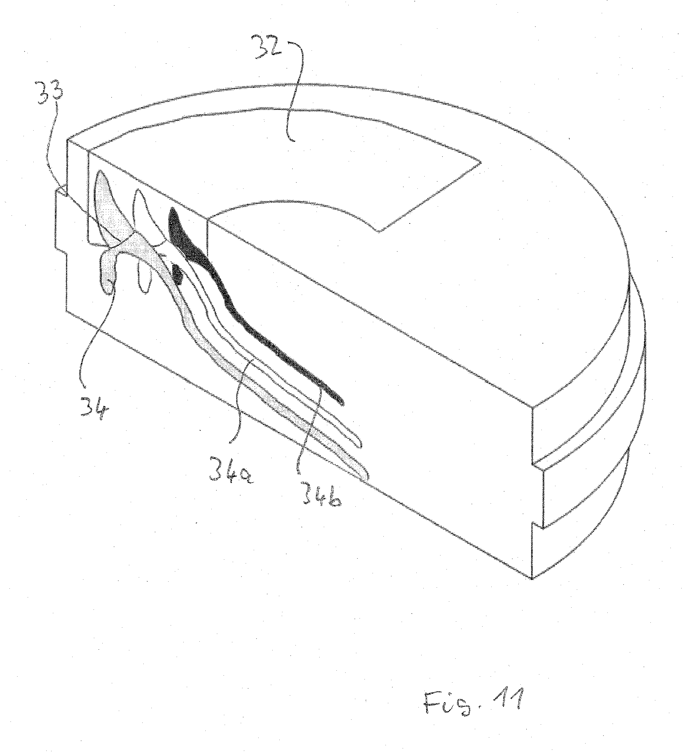

Fig. 11 zeigt einen Schnitt entsprechend Fig. 10, wobei verschiedene Prothese 34, 34a und 34b in alternativer Größendimensionierung dargestellt sind. Es ist ersichtlich, dass der Rohling sowohl zur Okklusal-/lnzisalseite, also im Bereich des Zahnbogens 32, als auch im zentralen Bereich nach unten hin, genügend Freiraum für die größten vorkommenden Kombinationen aus Prothesenbasen und Zähnen belässt, wobei es sich versteht, dass die Abmessungen des Rohlings der Steuervorrichtung als Randbedingung bekannt sind.

## Patentansprüche

1. Dentaler Prothesenrohling, der aus einem fleischfarbenen Material (10) und einem
zahnfarbenen Material (32), insbesondere je auf Kunststoffbasis, aufgebaut ist, wobei das zahnfarbene Material (32) des Rohlings eine Größe aufweist, die größer als ein menschlicher Zahnbogen ist, wobei das fleischfarbene Material (10) und das zahnfarbene
Material (32) durch Kleben oder Polymerisieren intensiv miteinander verbunden sind, **dadurch gekennzeichnet, dass** die Grenzfläche (33) zwischen den Materialien eine Wellenform (14) aufweist, welche - im Zahnbogenverlauf betrachtet - wellenförmig und - in vestibulärer Richtung betrachtet - strahlenförmig ausgebildet ist, wobei sich die Strahlen zu einem Mittelpunkt (17) erstrecken.

2. Dentaler Prothesenrohling nach Anspruch 1, **dadurch gekennzeichnet, dass** die Wellenform (14) von innen nach außen, also in vestibulärer Richtung, strahlenförmig aufgeweitet ist, und die Wellenform (14) Wellentäler (20) und Wellenberge (18) aufweist, und dass die Wellentäler (20) und die Wellenberge (18), über den Verlauf des Zahnbogens betrachtet, innen und außen, also über den Verlauf der Strahlenform, an der gleichen Winkelposition angeordnet sind.

3. Dentaler Prothesenrohling nach einem der vorhergehenden Ansprüche, **dadurch gekennzeichnet, dass** das zahnfarbene Material (32) bogenförmig unter Bildung eines Zahnbogen-Rohlings angeordnet ist, wobei die Breite des Bogens (16) zwischen 0,8 cm und 4 cm beträgt und insbesondere zwischen 1,2 cm und 3 cm liegt.

4. Dentaler Prothesenrohling nach einem der vorhergehenden Ansprüche, **dadurch gekennzeichnet, dass** die Wellenform (14) im Wesentlichen als Sinuswelle oder als Kettenlinie oder als Mischform dieser mit sich insbesondere über den Verlauf änderner oder über den Verlauf schwankender Amplitude und/oder Periode ausgebildet ist, wobei es insbesondere vorgesehen ist, dass die wellenförmige Ausgestaltung im Wesentlichen als Kettenlinie ausgebildet ist, wobei den Zahnzwischenräumen Aufhängpunkte der Kette entsprechen, die jedoch im Unterschied zur reinen Kettenlinie abgeflacht und/oder abgerundet sind.

5. Dentaler Prothesenrohling nach einem der Ansprüche 2 bis 4, **dadurch gekennzeichnet, dass** die Höhe und/oder Breite der Wellenberge (18) des zahnfarbenen Materials (32) von innen nach außen, also in vestibulärer Richtung, zunimmt, und dass die Grenzfläche zwischen den Materialien im Zahnbogenverlauf kettenlinienförmig - entsprechend dem natürlichen Zahnfleischsaum - ausgebildet ist.

6. Dentaler Prothesenrohling nach einem der vorhergehenden Ansprüche, **dadurch gekennzeichnet, dass** das fleischfarbene Material (10) des Protheserohlings als Scheibe ausgebildet ist, insbesondere in einem Standarddurchmesser wie etwa 98 mm, und dass es an seiner Oberseite ein Muster aus sich strahlenförmig erstreckenden Wellenlinien aufweist, das die exakte Negativform des entsprechenden Musters des zahnfarbenen Materials (32) bildet.

7. Dentaler Prothesenrohling nach einem der vorhergehenden Ansprüche, **dadurch gekennzeichnet, dass** das zahnfarbene Material (32) formschlüssig passend zum fleischfarbenen Material (10) ausgebildet und insbesondere mindestens in einer Vertiefung in dem fleischfarbenen Material aufnehmbar ist, und dass der Prothesenrohling aus bis zu fünf Teilen aus fleischfarbenen Material (10) und zahnfarbenen Material (32), oder aus einem Material mit gegenüber diesen Materialien unterschiedlicher Festigkeit besteht, die für das miteinander Zusammenfügen bestimmt sind, oder alternativ das zahnfarbene Material (32) und das fleischfarbene Material (10) als einstückiger Prothesenrohling - ggf. in Kombination mit dem Material mit unterschiedlicher Festigkeit - ausgebildet sind, wobei insbesondere ein Material höherer Festigkeit wie Metall in einer Aussparung des fleischfarbenen Materials (10) aufgenommen ist.

8. Dentaler Protheserohling nach einem der vorhergehenden Ansprüche , **dadurch gekennzeichnet, dass** das zahnfarbene Material (32) in eine mit einer wellenförmigen Profilierung versehene Ausnehmung (12) des fleischfarbenen Materials (10) gegossen und polymerisiert ist.

9. Dentaler Prothesenrohling nach einem der vorhergehenden Ansprüche, **dadurch gekennzeichnet, dass** das zahnfarbene Material (32) mehrschichtig ausgebildet ist, wobei insbesondere dem fleischfarbenen Material (10) zugewandte Schichten oder Bereiche dunkler
und/oder opaker und von diesen abgewandten Bereiche heller und/oder transluzenter ausgebildet sind, wobei insbesondere Grenzflächen zwischen Schichten, die dem fleischfarbenen Material (10) zugewandt sind, wellen/strahlenförmig sind, jedoch weniger stark ausgeprägt als die Grenzfläche zwischen dem zahnfarbenen Material (32) und dem fleischfarbenen Material (10).

10. Dentaler Prothesenrohling nach einem der vorhergehenden Ansprüche, **dadurch gekennzeichnet, dass** dieser eine Scheibe oder ein Element aus zahnfarbenen Material (32) aufweist, wobei die Scheibe oder das Element in Strahlenrichtung nach radial außen eine zunehmende Höhe, entsprechend einer größeren Zahnhöhe, aufweist.

11. Verfahren zur Herstellung einer dentalen Prothese (30, 34) aus einem Prothesenrohling gemäß einem der Ansprüche 1 bis 10, wobei die Prothese von einer CAD/CAM-Vorrichtung hinsichtlich ihrer Raumform festgelegt wird, welche CAD/CAM-Vorrichtung eine Steuervorrichtung aufweist, wobei die Steuervorrichtung basierend auf der von einer Scannvorrichtung erfassten Mundsituation des Patienten individuelle Zahnformen, die Rotation und/oder die Angulation der Zähne und die Form der Prothesenbasis festlegt, und die Steuervorrichtung die radiale Lage des Zahnbogens der Prothese festlegt, und wobei der per CAD festgelegte Zahnbogen in einem subtraktiven Verfahren zusammen mit der Prothesenbasis aus dem Prothesenrohling erzeugt wird.

## Claims

1. A dental prosthesis blank which is composed of a flesh-colored material (10) and a tooth-colored material (32), in particular on a respective plastic base, the tooth-colored material (32) of the blank being of a size which is larger than a human dental arch, the flesh-colored material (10) and the tooth-colored material (32) being intensively bonded to each other by bonding or polymerization, **characterized in that** the interface (33) between the materials has an undulating shape (14) which is undulating when viewed in the dental arch direction and radiating when viewed in the vestibular direction, the rays extending to a center point (17).

2. The dental prosthesis blank according to claim 1, **characterized in that** the undulating shape (14) is radially widened from the inside to the outside, i.e. in the vestibular direction, and the undulating shape (14) has wave troughs (20) and wave crests (18), and **in that** the wave troughs (20) and the wave crests (18), as viewed over the course of the dental arch, are arranged at the same angular position on the inside and outside, i.e. are arranged at the same angular position over the course of the beam shape.

3. The dental prosthesis blank according to one of the preceding claims, **characterized in that** the tooth-colored material (32) is arranged in the shape of an arch to form a dental arch blank, the width of the arch (16) being between 0.8 cm and 4 cm, and in particular between 1.2 cm and 3 cm.

4. The dental prosthesis blank according to one of the preceding claims, **characterized in that** the undulating shape (14) is essentially formed as a sine wave or as a catenary or as a mixed form thereof, having an amplitude and/or period especially changing over the course or fluctuating over the course, wherein it is especially provided for the undulating shape to be essentially formed as a catenary, the interdental spaces corresponding to suspension points of the catenary which suspension points are flattened and/or rounded, contrary to the pure catenary.

5. The dental prosthesis blank according to one of the claims 2 to 4, **characterized in that** the height and/or width of the wave crests (18) of the tooth-colored material (32) increases from the inside to the outside, i.e. in the vestibular direction, and **in that** the interface between the materials the is formed in the shape of a catenary across the dental arch course - corresponding to the natural gingival margin.

6. The dental prosthesis blank according to one of the preceding claims, **characterized in that** the flesh-colored material (10) of the prosthesis blank is formed as a disc, especially having a standard diameter such as about 98 mm, and **in that**, on the upper side thereof, it has a pattern of radially extending undulating lines forming the exact negative shape of the corresponding pattern of the tooth-colored material (32).

7. The dental prosthesis blank according to one of the preceding claims, **characterized in that** the tooth-colored material (32) is formed in a form-fitting manner to match the flesh-colored material (10) and, in particular, is receivable at least in a depression in the flesh-colored material, and **in that** the prosthesis blank consists of up to five parts of flesh-colored material (10) and tooth-colored material (32), or of a material having different strength as compared to these materials, which are intended to be joined together, or alternatively the tooth-colored material (32) and the flesh-colored material (10) is formed as an integral prosthesis blank - eventually in combination with the material having different strength, wherein especially a material having higher strength such as metal is accommodated in a recess of the flesh-colored material (10).

8. The dental prosthesis blank according to one of the preceding claims, **characterized in that** the tooth-colored material (32) is cast and polymerized in a recess (12) of the flesh-colored material (10) provided with an undulating profiling.

9. The dental prosthesis blank according to one of the preceding claims, **characterized in that** the tooth-colored material (32) is formed in multiple layers, wherein layers or regions facing the flesh-colored material (10) are especially made darker and/or more opaque and regions facing away therefrom are made lighter and/or more translucent, wherein especially interfaces between layers facing the flesh-colored material (10) are undulating/radiating, but are formed less pronounced than the interface between the tooth-colored material (32) and the flesh-colored material (10).

10. The dental prosthesis blank according to any of the preceding claims, **characterized in that** it comprises a disc or element of tooth-colored material (32), said disc or element having an increasing height in the radially outward direction, corresponding to a greater tooth height.

11. A method for producing a dental prosthesis (30, 34) from a prosthesis blank according to any one of claims 1 to 10, wherein the prosthesis is determined by a CAD/CAM device with regard to its spatial shape, which CAD/CAM device comprises a control device, wherein the control device, based on the patient's oral situation detected by a scanning device, determines individual tooth shapes, the rotation and/or the angulation of the teeth and the shape of the denture base, and the control device determines the radial position of the dental arch of the denture, and wherein the dental arch determined by CAD is produced together with the denture base from the denture blank in a subtractive process.

## Revendications

1. Lingotin de prothèse dentaire constituée d'un matériau de la couleur de la chair (10) et un matériau de la couleur des dents (32), en particulier chacun sur une base plastique, où le matériau (32) du lingotin de la couleur des dents, présente une taille qui est supérieure à celle d'une arcade dentaire humaine, où le matériau de la couleur de la chair (10) et le matériau de la couleur des dents (32) sont intensivement liés entre eux par collage ou polymérisation, **caractérisé en ce que** l'interface (33) entre les matériaux présente une forme ondulée (14) qui - vue sur le parcours de l'arcade dentaire - est en forme de vague et - vue dans la direction vestibulaire - est en forme de rayons, où les rayons s'étendent vers un point central (17).

2. Lingotin de prothèse dentaire selon la revendication 1, **caractérisé en ce que** la forme ondulée (14) est élargie radialement de l'intérieur vers l'extérieur, c'est-à-dire dans la direction vestibulaire, et la forme ondulée (14) présente des creux d'onde (20) et des crêtes d'onde (18), et **en ce que** les creux d'onde (20) et les crêtes d'onde (18), vues sur le parcours de l'arcade dentaire, à l'intérieur et à l'extérieur, c'est-à-dire sur le parcours de la forme en rayons, sont disposées à la même position angulaire.

3. Lingotin de prothèse dentaire selon l'une des revendications précédentes, **caractérisé en ce que** le matériau de la couleur des dents (32) est disposé en forme d'arc pour former un lingotin d'arcade dentaire, où la largeur de l'arc (16) est comprise entre 0,8 cm et 4 cm, et en particulier entre 1,2 cm et 3 cm.

4. Lingotin de prothèse dentaire selon l'une des revendications précédentes, **caractérisé en ce que** la forme ondulée (14) est conçue sensiblement comme une onde sinusoïdale ou comme une chainette ou comme une forme mixte de celles-ci avec, en particulier, une amplitude et/ou une période changeant ou fluctuant sur son parcours, où il est prévu en particulier que la configuration ondulatoire est conçue essentiellement comme une chainette, où les espaces entre les dents correspondent à des points de suspension de la chaînette qui, cependant, contrairement à la chaînette pure, sont aplaties et/ou arrondies.

5. Lingotin de prothèse dentaire selon l'une des revendications 2 à 4, caractérisé en ce la hauteur et/ou la largeur des crêtes (18) du matériau de la couleur des dents (32) augmente de l'intérieur vers l'extérieur, c'est-à-dire dans la direction vestibulaire, et en ce que l'interface entre les matériaux au cours de l'arcade dentaire est en forme de chaînette - correspondant au rebord gingival naturel.

6. Lingotin de prothèse dentaire selon l'une des revendications précédentes, **caractérisé en ce que** le matériau de la couleur de la chair (10) du lingotin de prothèse est conçu comme un disque en particulier dans un diamètre standard d'environ 98 mm, et **en ce qu'**il présente sur sa face supérieure un motif de lignes ondulées s'étendant en forme de rayons qui forme la forme négative exacte du motif correspondant du matériau de la couleur des dents (32).

7. Lingotin de prothèse dentaire selon l'une des revendications précédentes, caractérisé en ce le matériau de la couleur des dents (32) est formé en correspondance de forme avec le matériau de la couleur de la chair 10) et en particulier peut être logé dans au moins un évidement du matériau de la couleur de la chair, et en ce que le lingotin de la prothèse est composée de jusqu'à cinq pièces en matériau de la couleur de la chair (10) et en matériau de la couleur des dents (32), ou d'un seul matériau ayant une rigidité différente par rapport à ces matériaux, qui sont destinés à être assemblés, ou alternativement le matériau de la couleur des dents (32) et le matériau de la couleur de la chair (10) sont conçus comme lingotin de prothèse d'une seule pièce - éventuellement en combinaison avec le matériau ayant une rigidité différente - où en particulier un matériau d'une rigidité plus élevée, tel que du métal, est logé dans un évidement du matériau de la couleur de la chair (10).

8. Lingotin de prothèse dentaire selon l'une des revendications précédentes, **caractérisé en ce que** le matériau de la couleur des dents (32) est coulé et polymérisé dans un évidement (12) du matériau de la couleur de la chair (10) pourvu d'une surface ondulée.

9. Lingotin de prothèse dentaire selon l'une des revendications précédentes, **caractérisé en ce que** le matériau de la couleur des dents (32) est formé en plusieurs couches, où en particulier les couches ou les régions faisant face au matériau de la couleur de la chair (10) sont formées plus foncées et/ou plus opaques et les régions qui en sont éloignées sont formées plus claires et/ou plus translucides, où en particulier les interfaces entre les couches faisant face au matériau de la couleur de la chair (10) sont ondulées/rayonnées, mais moins prononcées que l'interface entre le matériau de la couleur des dents (32) et le matériau de la couleur de la chair (10).

10. Lingotin de prothèse dentaire selon l'une des revendications précédentes, **caractérisé en ce que** celui-ci comprend un disque ou un élément en matériau de la couleur de la dent (32), où le disque ou l'élément présente une hauteur croissante dans la direction des rayons radialement vers extérieure, correspondant à une hauteur de dent plus élevée.

11. Procédé de fabrication d'une prothèse dentaire (30, 34) à partir d'une lingotin de prothèse selon l'une des revendications 1 à 10, où la prothèse est déterminée par un dispositif de CFAO en ce qui concerne sa forme spatiale, lequel dispositif de CFAO comprend un dispositif de commande, où le dispositif de commande, sur la base de la situation buccale du patient détectée par un dispositif de balayage, détermine des formes de dents individuelles, la rotation et/ou l'angulation des dents et la forme de la base de la prothèse, et le dispositif de commande détermine la position radiale de l'arcade dentaire de la prothèse, et où l'arcade dentaire déterminée par CAO est produite dans un processus soustractif avec la base de la prothèse à partir du lingotin de la prothèse.
